# EUROPEAN PATENT APPLICATION

(11) **EP 4 147 650 A1**
(43) Date of publication of application: **15.03.2023**
(21) Application number: 22194531.4
(22) Date of filing: 08.09.2022
(51) Int. Cl.: A61B 17/12

(54) **IMPLANTABLE MEDICAL DEVICE DETACHMENT SYSTEM WITH SPLIT TUBE AND CYLINDRICAL COUPLING**

(30) Priority: 09.09.2021 US 202117470009
(71) Applicant: DePuy Synthes Products, Inc., Raynham, MA 02767 (US)
(72) Inventor: BLUMENSTYK, David, Raynham, 02767 (US); SOTO DEL VALLE, Ariel, Raynham, 02767 (US); SOLAUN, Daniel, Raynham, 02767 (US); LORENZO, Juan, Raynham, 02767 (US)
(74) Representative: Carpmaels & Ransford LLP

(57) **Abstract**

A method of constructing a detachment system for delivering an implantable medical device to a target location of a body vessel is presented. The method includes forming a compressible portion on a distal tube, engaging an implantable medical device with an engagement system, extending the engagement system through the distal tube such that the implantable medical device is distal of a distal end of the distal tube, applying a force to the engagement system to compress the compressible portion to a compressed state, fixing the engagement system to the distal tube to maintain the compressed state of the compressible portion, and joining a proximal end of the distal tube to a distal end of a proximal tube. The engagement system can include a loop wire that is fixed to the distal tube and engages the medical device.

## Description

### Cross-Reference to Related Applications

The present application is a continuation-in-part application of U.S. Patent Application No. 17/064,907 filed October 7, 2020, which is a divisional application of U.S. Patent Application No. 15/850,993 filed December 21, 2017, which issued as U.S. Patent No. 10,806,462 on October 20, 2020, the contents of which are incorporated by reference as if set forth in its entirety herein.

### Field of the Invention

This invention generally relates to interventional medical device systems that are navigable through body vessels of a human subject. More particularly, this invention relates to detachment systems for deploying an implantable medical device to a target location of a body vessel and methods of using the same.

### Background

The use of catheter delivery systems for positioning and deploying therapeutic devices, such as dilation balloons, stents, and embolic coils, in the vasculature of the human body has become a standard procedure for treating endovascular diseases. It has been found that such devices are particularly useful in treating areas where traditional operational procedures are impossible or pose a great risk to the patient, for example in the treatment of aneurysms in cranial blood vessels. Due to the delicate tissue surrounding cranial blood vessels, especially for example brain tissue, it is very difficult and often risky to perform surgical procedures to treat defects of the cranial blood vessels. Advancements in catheter deployment systems have provided an alternative treatment in such cases. Some of the advantages of catheter delivery systems are that they provide methods for treating blood vessels by an approach that has been found to reduce the risk of trauma to the surrounding tissue, and they also allow for treatment of blood vessels that in the past would have been considered inoperable.

Typically, these procedures involve inserting the distal end of a delivery catheter into the vasculature of a patient and guiding it through the vasculature to a predetermined delivery site. A vascular occlusion device, such as an embolic coil, is attached to the end of a delivery member which pushes the coil through the catheter and out of the distal end of the catheter into the delivery site. Some of the problems that have been associated with these procedures relate to ensuring the complete release and deployment of the coil. For example, U.S. Pat. No. 5,250,071 to Palermo, which is hereby incorporated herein by reference, describes a detachment system whereby interlocking clasps of the system and the coil are held together by a control wire. The control wire is moved proximally to disengage the clasps from each other. However, the system does not include any positive means for separating the disengaged clasps from each other, so merely retracting the control wire does not ensure release and deployment of the coil. Numerous other detachment systems currently in use suffer from similar problems.

In addition, U.S. Pat. No. 8,062,325, which is hereby incorporated herein by reference, discloses a single tubular carrier to deliver and deploy the vascular occlusion device, but has only a single compressible section. Therefore, a need remains for a more rapid release detachment system or method that can ensure release and deployment of an implantable medical device. Further advantages could be realized with a detachment system or method incorporating a simple and inexpensive locking and deployment system.

### Summary

A detachment system delivers an implantable medical device to a target location of a body vessel with a generally hollow distal tube. The distal tube has a proximal end, a distal end, and a compressible portion of the distal tube itself axially movable from a compressed condition to an elongated condition, between the proximal and distal ends. Also includes is a generally hollow proximal tube having a proximal end and a distal end, a coupling disposed between the proximal end of the distal tube and the distal end of the proximal tube, joining the proximal and distal tubes, and an engagement system engaging and deploying the implantable medical device engaged at the distal end of the distal tube. The engagement system moves the compressible portion to the compressed condition when engaging the implantable medical device and deploys the implantable medical device and releases the compressible portion to the elongated condition.

In another example, the engagement system can be removably fixed to the proximal end of the distal tube when engaging the implantable medical device to maintain the compressed condition. Also, the engagement system can be removably fixed to the proximal end of the proximal tube when engaging the implantable medical device.

An example of the engagement system has a locking member and a loop wire. When the loop wire interacts with the locking member to engage the implantable medical device, a force on the loop wire moves the compressible portion to the compressed condition, and the loop wire is welded to the proximal end of the distal tube to removably fix the engagement system. A force on the locking member releases the loop wire, disengages the implantable medical device, and allows the compressible portion to return the elongated condition.

Other examples have the compressible portion of the distal tube as a spiral-cut portion of the distal tube. The compressible portion can be adapted to deploy the implantable medical device engaged by the engagement system when the compressible portion moves to the elongated condition. Further, the compressible portion of the distal tube is adapted to automatically/resiliently move to the elongated condition when the engagement system is disengaged from the implantable medical device. The proximal tube can also include a flexible portion of the proximal tube itself, between the proximal and distal ends which is flexible, and the distal tube can comprise a flexible portion of the distal tube itself, between the proximal end and the compressible portion, which is flexible.

A further example has the proximal tube partially overlapping the coupling, the distal tube partially overlapping the coupling, and a gap formed on the coupling between the proximal tube and the distal tube includes a weld band to weld the coupling to the proximal tube and the distal tube. In an example, the coupling is radiopaque.

A method of detaching an implantable medical device, using the examples above can include the steps of forming a compressible portion on the distal tube between the proximal and distal ends, engaging the implantable medical device with an engagement system, applying a force to the engagement system to compress the compressible portion, fixing the engagement system to the distal tube to maintain a compressed state, and joining the distal tube and proximal tube together using the coupling. As above, the engagement system can be removably fixed to the proximal end of the distal tube.

The detachment method example can further have the step of removably fixing the engagement system to the proximal end of the proximal tube when engaging the implantable medical device. The engagement step can include the step of using the loop wire with the locking member to engage the implantable medical device; and the applying step further comprises the step of applying force to the loop wire to move the compressible portion to the compressed condition. Other example steps include applying a force on the locking member, disengaging the implantable medical device, and allowing the compressible portion to return the elongated condition.

Examples of the forming step can include the step of spiral-cutting a portion of the distal tube and the further have the step of deploying the implantable medical device engaged by moving the compressible portion to the elongated condition. Additionally, the compressible portion of the distal tube can be adapted to automatically/resiliently move to the elongated condition when the engagement system is disengaged from the implantable medical device.

Further, the joining step further has the steps of partially overlapping the proximal tube over the coupling, partially overlapping the distal tube over the coupling, forming a gap on the coupling between the proximal tube and the distal tube comprising a weld band, and welding the coupling to the proximal tube and the distal tube at the weld band.

### Brief Description of the Drawings

The above and further aspects of this invention are further discussed with reference to the following description in conjunction with the accompanying drawings, in which like numerals indicate like structural elements and features in various figures. The drawings are not necessarily to scale, emphasis instead being placed upon illustrating principles of the invention. The figures depict one or more implementations of the inventive devices, by way of example only, not by way of limitation.
Figure 1A is an exploded view of an example of the detachment system of the present invention with the medical device partially disengaged;
Figure 1B is a magnified view of Figure 1A;
Figure 2 is an exploded view of an example of the detachment system of the present invention with the medical device engaged;
Figure 3A is a side perspective view of an example of a loop wire according to an example;
Figure 3B is a plan view of an example of a loop wire according to another example;
Figure 4 is a front perspective detail view of an opening of the loop wire in an up-turned condition in an alternate example;
Figure 5A is an exploded view of an example of the detachment system of the present invention with the medical device engaged and the loop wire secured;
Figure 5B is a magnified view of the loop wire secured to the distal tube;
Figure 6 is a plan view of the proximal and distal tubes overlapping the coupling;
Figure 7 is a plan view of the proximal and distal tubes welded to the coupling;
Figure 8 illustrates the proximal weld at the small tube;
Figure 9 illustrates the fluoroscopic view of an example of the detachment system;
Figure 10 illustrates an example method of forming the detachment system of the present invention;
Figures 11A-11D illustrate the medical device being detached with a partial cross-section;
Figure 12 is a side view of an example of the distal tube in the compressed and expanded state;
Figure 13 is a front-side perspective view of an example of the medical device being detached;
Figure 14 is a plan view of the first flexible portion and second flexible portion of the distal tube;
Figure 15 is a magnified view of Figure 14;
Figure 16 is a side view of an example distal tube in a tortuous path in a vasculature; and
Figures 17A-17D illustrate example positions of the distal tube during engagement and detachment.

### Detailed Description

The figures illustrate a generally hollow or tubular structure according to the present invention. When used herein, the terms "tubular" and "tube" are to be construed broadly and are not limited to a structure that is a right cylinder or strictly circumferential in cross-section or of a uniform cross-section throughout its length. For example, the tubular structure or system is generally illustrated as a substantially right cylindrical structure. However, the tubular system may have a tapered or curved outer surface without departing from the scope of the present invention.

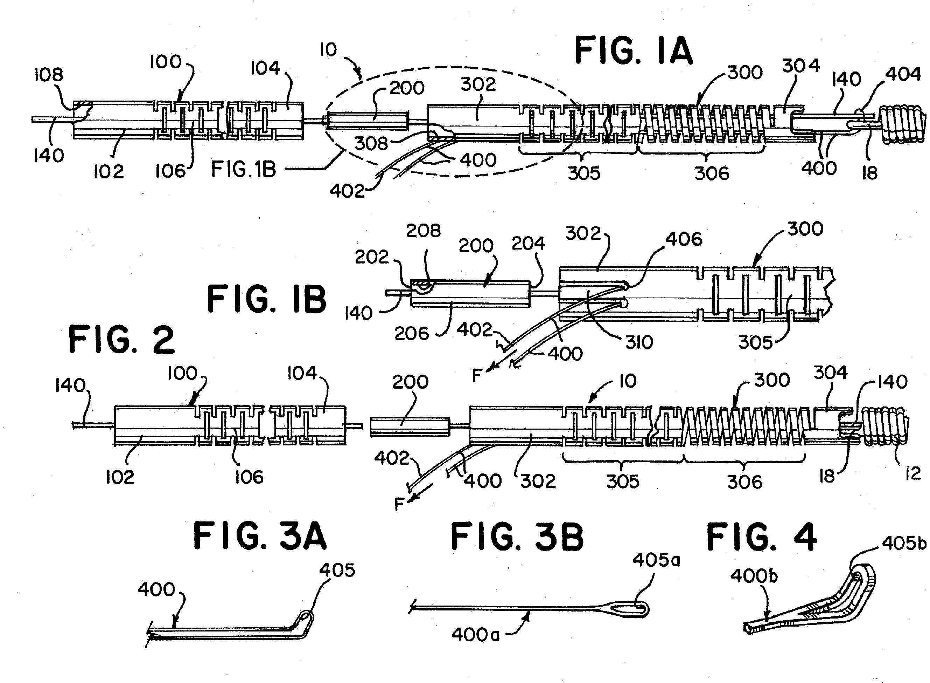

An example of a detachment system 10 of the present invention, as illustrated in Figures 1A, 1B, and 2, can have a proximal elongated delivery hypotube assembly 100, an intermediate coupling 200, and a distal delivery tube 300. An implantable medical device 12 is engaged at one end of the distal delivery tube 300. The implantable medical device 12 can be an embolic coil, but it will be appreciated that virtually any implantable medical device 12 may be delivered and deployed by the detachment system 10 according to the present invention. The medical device 12 is engaged to the system using a locking member 140 and a loop wire 400. The medical device 12 has a locking portion 18 to interface with an engagement system 140, 400.

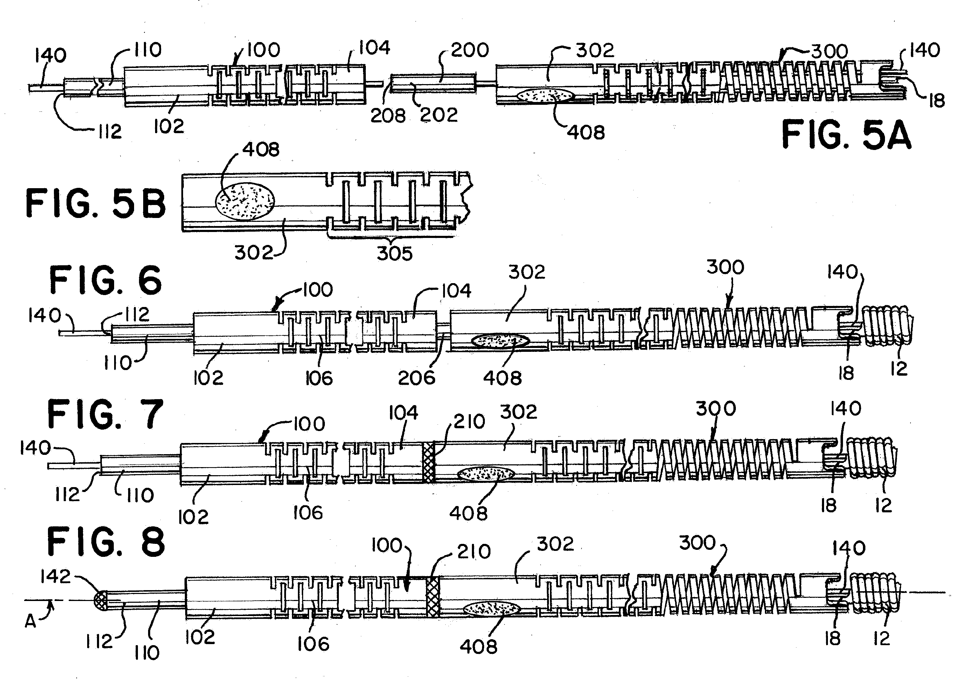

The proximal delivery tube 100 can have a proximal end portion 102, distal end portion 104, and a flexible portion 106 in between. The proximal delivery tube 100 forms an axial lumen 108 therein. The proximal end 102 engages with a smaller diameter tube 110 (see Figures 5A, 6-8) along the axial lumen 108. The distal delivery tube 300 can have a proximal end portion 302, distal end portion 304, and between the two, a compressible portion 306. In one example, the compressible portion 306 can be closer to the distal end portion 304, and between the proximal end portion 302 and the compressible portion 306 can be a flexible portion 305. The distal delivery tube 300 forms an axial lumen 308 therein.

The delivery tubes 100, 300 can be made of a biocompatible material, such as stainless steel. The tubes 100, 300 can typically have a diameter of between about 0.010 inch and about 0.018 inch, a preferred tube having a diameter of approximately 0.0145 inch. These examples of tube size are suitable for delivering and deploying embolic coils to target locations, typically aneurysms, within the neurovasculature. Differently sized tubes 100, 300 comprised of other materials may be useful for different applications and are within the scope of the present invention.

The flexible portions 106, 305 allow the delivery tubes 100, 300 to bend and flex. This assists tracking the system 10 through the catheter and the tortuous path through the human vasculature. The flexible portions 106, 305 can be formed with interference spiral cuts. These cuts allow for gaps to permit bending but in one example, do not act as a spiral-cut spring. Thus, can bend and flex but do not compress.

The compressible portion 306 is axially adjustable between an elongated condition and a compressed condition. Preferably, the compressible portion 306 is formed from a spiral-cut portion of the tube 300, formed by a laser-cutting operation. However, any other arrangement allowing axial adjustment (e.g., a wound wire or spiral ribbon) is also suitable for use with detachment systems according to the present invention. Most preferably, the compressible portion 306 is in the elongated condition at rest and automatically or resiliently returns to the elongated condition from a compressed condition, unless otherwise constrained. The function of the compressible portion 306 is described in greater detail herein.

An example of the coupling 200 has a proximal section 202, a distal section 204, a weld band 206 between and an axial lumen 208 therein. The coupling 200 bridges both delivery tubes 100, 300, and can provide a radiopaque marking to assist in the alignment of the detachment system 10 in a delivery catheter while in clinical use. An example of the intermediate coupling 200 can be a marker band or coil segment.

Figures 3A, 3B, and 4 illustrate examples of the loop wire 400. The loop wire 400 can be relatively small, having the thickness of a hair in some embodiments, so it may be preferred for it to be entirely shielded by the distal end 304 of the distal delivery tube 300 to prevent damage from accidental contact. The loop wire 400 can be an elongated wire that is looped, as in Figure 3A. The loop wire 400a can also be a single elongated wire with an opening 405, as illustrated in Figure 3B. The opening 405 can be formed by loosely bending the loop wire 400a in half. In an alternative example, the loop wire 400b comprises a flat ribbon defining an opening 405a at a distal portion and the opening 405a can be in an up-turned condition suitable for engaging an end of the implantable medical device 12. An example of the loop wire 400, 400a, 400b can be elastically deformable to the up-turned condition such that it will return to the substantially flat condition when not otherwise constrained. The loop wire 400, 400a, 400b may be formed from of any of a number of materials, including nitinol and stainless steel.

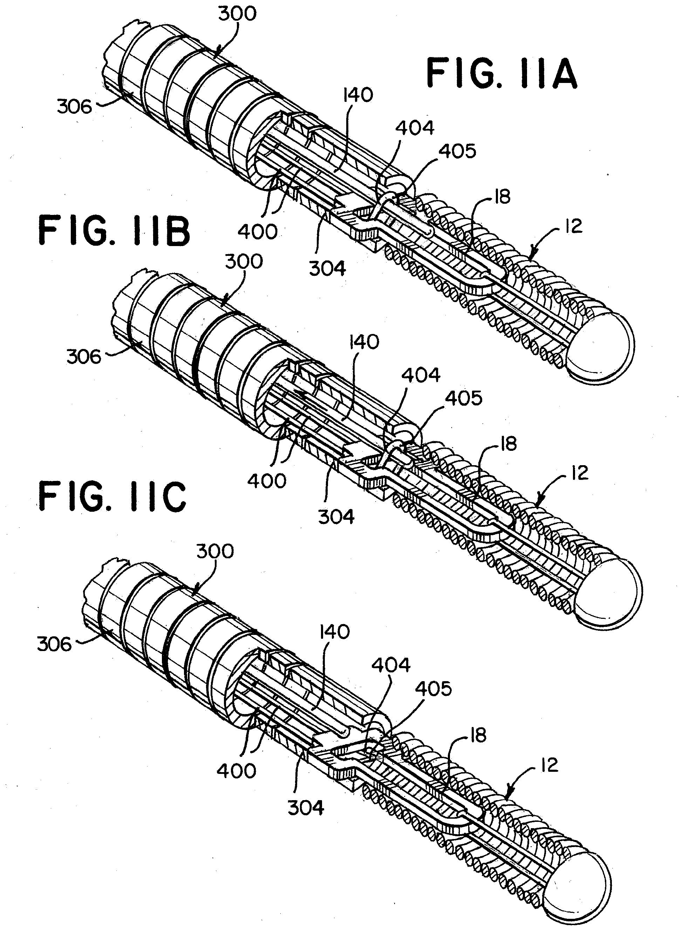

To load the detachment system 10, the locking member 140 is inserted axially within the lumens 108, 208, 308 of both tubes 100, 300 and the coupling 200. A distal end 404 of the loop wire 400 is inserted into the distal delivery tube 300 through an anchor portion 310 located on the proximal end 302 of the distal tube 300 and passed through the lumen 308 to the distal end 304. The distal end of the loop wire 404 can then be looped to form the opening 405. The opening 405 is passed through the locking portion 18 and the locking member 140 is passed through the opening 405 to engage the medical device 12. See, Figures 1A and 11A.

The loop wire 400 is pulled taught at a proximal end of the loop wire 402 and continued force F compresses the compressible portion 306. The amount of compression can be controlled by the amount of force F applied to the proximal end 402 of loop wire 400 after the medical device 12 is mounted on the distal end 304 of the distal tube 300. Figures 2 and 11A illustrate the mounted medical device 12 and the distal tube 300 in a compressed state. Once the distal tube 300 is compressed the appropriate amount, the loop wire 400 is anchor welded 408 at wire weld point 406 (between the proximal 402 and distal 404 ends) to the proximal end 302 (i.e. behind the compressible portion 306) at or approximate to the anchor portion 310 of the distal delivery tube 300. See, Figures 5A and 5B. The level of compression of the distal delivery tube 300 is adjusted by varying the amount of force F on the loop wire 400 prior to securing the loop wire 400 in place with the anchor weld 408.

Figure 6 and 7 illustrate the joining of the proximal delivery tube 100 and the distal delivery tube 300 using the coupling 200. Figure 6 illustrates the distal end 104 of the proximal tube 100 being pulled toward and overlapping the proximal end 202 of the coupling 200. Similarly, the proximal end 302 of the distal tube 300 is pulled toward and overlaps the distal end 204 of the coupling 200. The proximal and distal tubes 100, 300, in this example, do not come into contact, but leave the weld band 206 as a gap on the coupling 200. The two tubes 100, 300 are then circumferentially welded 210 together at the weld band 206 to form a unitary device 10. The intermediate coupling 200 bridges both delivery tubes 100, 300, as well as provides a radiopaque marking for alignment of the system 10 to a delivery catheter (not illustrated) while in clinical use.

Prior to the overlapping and welding of the two tubes and coupling, 100, 200, 300, the locking member 140 (as discussed above) is pulled through the coupling lumen 208 and the proximal tube lumen 108 through to the small tube 110. At a proximal opening 112 in the small tube 110, opposite the proximal end 102 of the proximal tube 100, the locking member 140 is welded 142 to the small tube 110. This is illustrated in Figure 8.

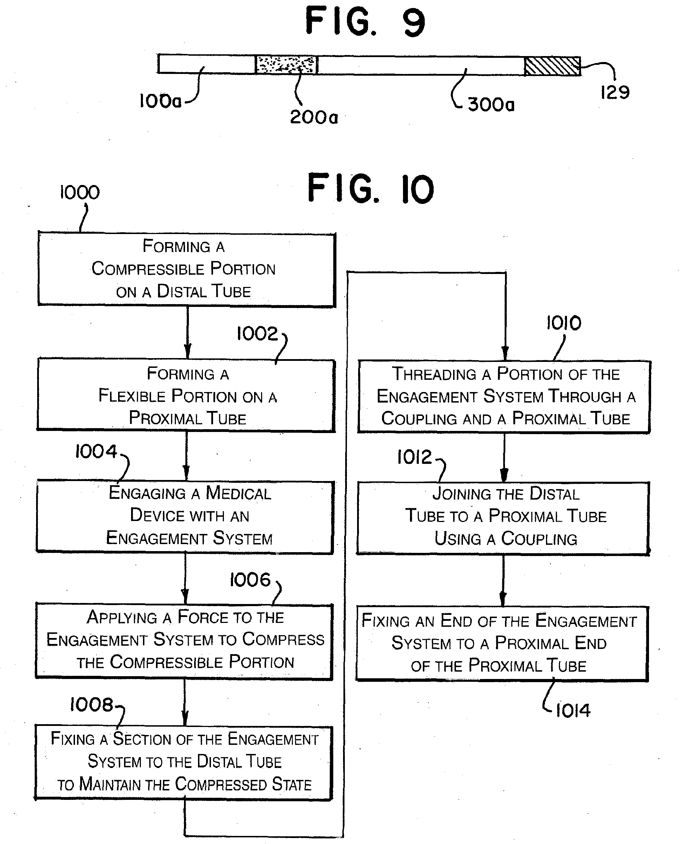

Figure 9 illustrates the detachment system 10 in a fluoroscopic view. Given that the coupling 200 and the medical device 12 typically are made of or have radiopaque markings, it allows for a view of the proximal 100a and distal 300a tubes having a different contrast from the coupling 200a or the medical device 12a. This provides visual feedback to indicate when the device 12a has been released (to be discussed further below).

Figure 10 illustrates an example of a method of assembling the detachment system 10. The method includes forming the compressible portion 306 on the distal tube 300 (step 1000) and forming the flexible portion 106 on the proximal tube 100 (step 1002). Step 1002 can also include forming the flexible portion 305 on the distal tube 300. The compressible portion 306 can be formed by spiral cutting the distal tube 300 or by any other means to form a tube that can be compressed and then return to its uncompressed state quickly. The flexible portion 106 of the proximal tube 100 can be interference cut or by any other means to increase the flexibility of the proximal tube 100. Once at least the distal tube 300 is ready, the medical device 12 can be engaged with an engagement system 140, 400 (step 1004) and a force F can be applied to the engagement system 140, 400 to compress the compressible portion 306 (step 1006). Here it is noted that while an example is presented above using the locking member 140 and the loop wire 400 as an engagement system, one of ordinary skill can realize different methods to secure the medical device 12 while still applying releasable force on the compressible portions 306 to be released when the engagement system 140, 400 is disengaged from the medical device 12. A section 406 of the engagement system 140, 400 is then engaged to the distal tube 300 to maintain the compressed state of the compressible portion 306 (step 1008). A portion of the engagement system 140, 400 is threaded through the coupling 200 and the proximal tube 100 (step 1010). The distal 300 and proximal tubes 100 are joined together using a coupling 200 (step 1012). Here, in this example, the ends 104, 302 of the tubes 100, 300 overlap the coupling 200 and all three are welded together 210. The end 144 of the engagement system 140, 400 can then be joined to a proximal end 102 of the proximal tube 100 (step 1014) to complete the device 10.

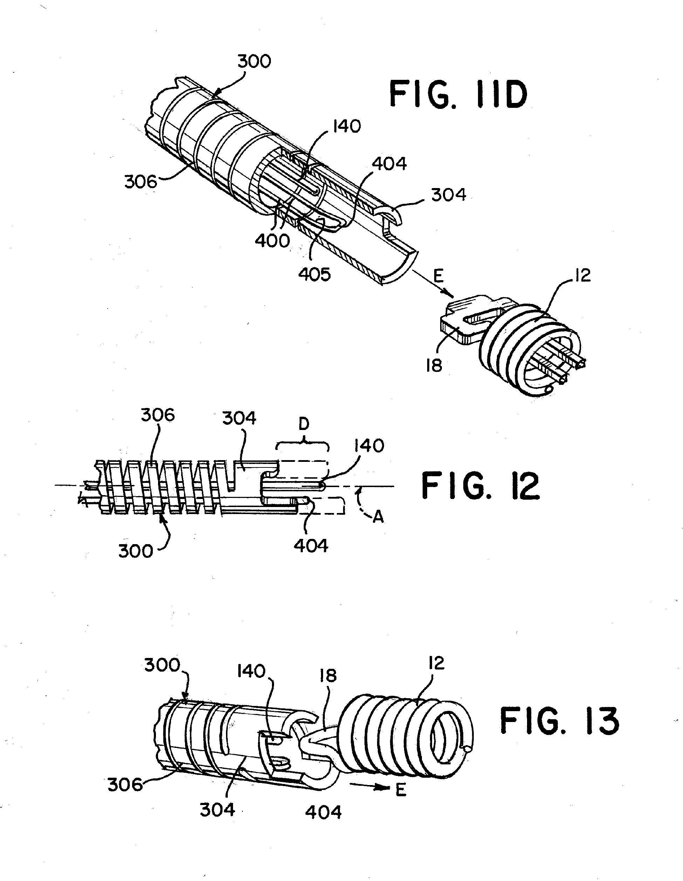

Turning to Figures 11A-11D, the detachment of the medical device 12 is illustrated in more detail. Figure 11A illustrates the engagement system 140, 400 locked into the locking portion 18 of the medical device 12. The loop wire 400 opening 405 can be placed through the locking portion 18. When the locking member 140 is put through the opening 405 the medical device 12 is now secure. Force F was previously applied to place the distal tube 300 in the compressed state. Figure 11B illustrates the locking member 140 being drawn proximally to begin the release sequence for the medical device 12. Figure 11C illustrates the instant the locking member 140 exits the opening 405 and is pulled free of the loop wire 400. The distal end 404 of the loop wire 400 falls away/returns to its preformed shape (as discussed above) and exits the locking portion 18. As can be seen, there is now nothing holding the medical device 12 to the detachment system 10. Figure 11D illustrates the end of the release sequence. Here, the compressible portion 306 has expanded/returned to its original shape and "sprung" forward. An elastic force E is imparted by the distal end 304 of the distal tube 300 to the medical device 12 to "push" it away to ensure a clean separation and delivery of the medical device 12.

Figure 12 shows the distal tube 300 illustrated without the medical device 12 but with the compressible portion 306 shortened in axial length to the compressed condition. In particular, a distance "D" is illustrated by which the distal tube 300 is axially foreshortened in moving the compressible portion 306 from the elongated condition to the compressed condition. This compression can occur along the axis A.

Figure 13 illustrates another view of the medical device 12 at the point of detachment. The locking member 140 has been pulled proximally so that it separated from the loop wire 400, allowing the medical device 12 to separate as the distal compressed portion 306 expands and furthers separates the medical device 12 from the delivery system 10. The arrow "E" denotes the elastic force "pushing" the medical device 12 away from the distal end 304 to assure a clean separation and delivery to the target site inside the patient. The elastic force E acts in the axis A of the lumen 308 and "pushes" the medical device 12 along the same axis A (see Figures 8 and 12).

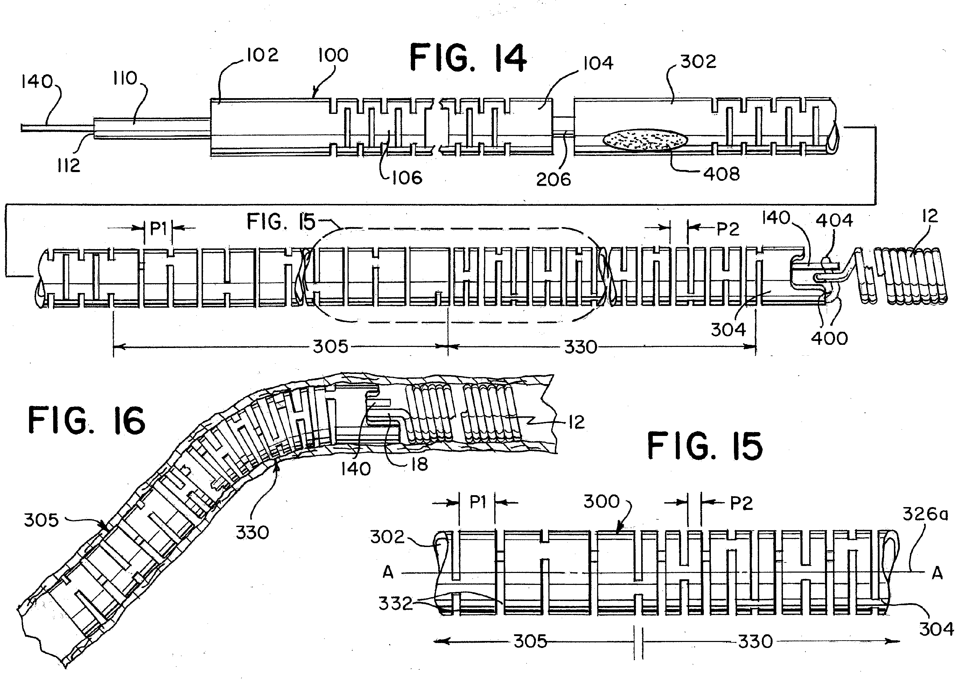

An example of a detachment system 10 of the present invention can have a proximal elongated delivery hypotube assembly 100, an intermediate coupling 200, and a distal delivery tube 300 having a first flexible portion 305 and a second flexible portion 330. As shown in Figure 14, an implantable medical device 12 is engaged at one end of the distal delivery tube 300. The implantable medical device 12 can be an embolic coil, but it will be appreciated that virtually any implantable medical device 12 may be delivered and deployed by the detachment system 10 according to the present invention. The medical device 12 is engaged to the system using a locking member 140 and a loop wire 400. The medical device 12 has a locking portion 18 to interface with an engagement system 140, 400.

Figure 15 shows a magnified view of Figure 14. To enable a controlled detachment of the implantable medical device 12, the distal delivery tube 300 can have a proximal end portion 302, distal end portion 304, and between the two, a second flexible or incompressible portion 330. In one example, the second flexible or incompressible portion 330 can be closer to the distal end portion 304, and between the proximal end portion 302. The second flexible or incompressible portion 330 can be adjacent a first flexible portion 305.

As illustrated in Figure 16, the flexible portions 106, 305, and 330 allow the delivery tubes 100, 300 to bend and flex laterally. This assists tracking the system 10 through the catheter and the tortuous path through the human vasculature. The flexible portions 106, 305, 330 can be formed with interference spiral cuts. These cuts allow for gaps to permit bending but in one example, do not act as a spiral-cut spring. Thus, can bend and flex but do not compress. The second flexible portion or incompressible portion 330 can have a different interference spiral cuttings than the first flexible portion 305. Preferably, the second flexible portion 330 is formed from modulated cut angles and spacing in interference spiral cutting compared to the first flexible portion 305 of the tube 300, which can be formed by a laser-cutting operation. The interference spiral cuts of the first and second flexible portions 305, 330 can include about 240-degree cut sections and about 20-degree uncut sections.

Referring back to Figure 15, the first flexible portion 305 can have a first pitch P1 and the second flexible portion 330 can have a second pitch P2 different than the first pitch P1. The first pitch P1 of the first flexible portion 305 can range from about 0.12 mm to about 0.18 mm, and preferable be about 0.15 mm. The second pitch P2 of the second flexible portion 330 can range from about 0.08 mm to about 0.12 mm, and preferably be about 0.1 mm.

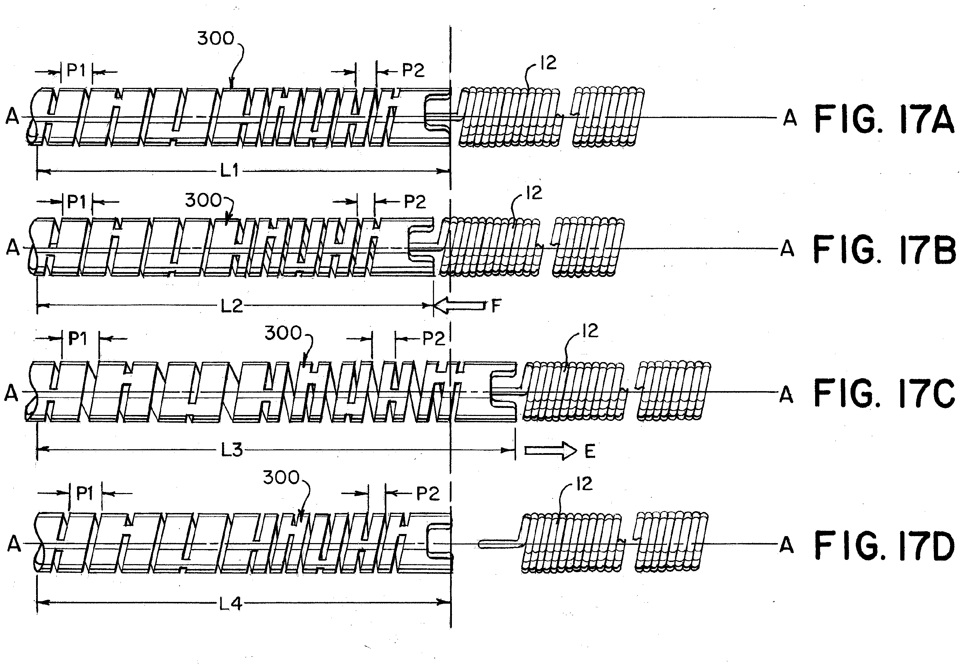

As shown in Figures 17A through 17D, the second flexible or incompressible portion 330 limits the amount of compression of the distal tube 300 required to hold the implantable medical device 12 securely while still able to deploy the implantable medical device 12 with control. Figure 17A shows the distal tube 300 measures a first length, L1 prior to the implantable medical device 12 being engaged. Figure 17B illustrates after the engagement system engages the implantable medical device 12, the distal tube 300 can measure a second length L2 smaller than the first length L1, where the first flexible portion 305 compresses to a different extent than the second flexible portion 330, for instance, the first flexible portion 305 compresses to a lesser extent than the second flexible portion 330. When the implantable medical device 12 is engaged and prior to deployment, the distal tube 300 can experience a force F of about 13-gram force (gf) to about 26 gf. The force F can compress the distal tube from about 0.15 mm to about 0.45 mm. Figure 17C shows the third length L3 that is a maximum length of the distal tube 300 upon deployment of the implantable device 12. The maximum extension length of the distal tube 300 upon deployment of the implantable device 12 can range from about 0.4 mm to about 0.8 mm from the compressed position of the second length L2. Additionally, the first flexible portion 305 extends to a different extent than the second flexible portion 330, for instance, the first flexible portion 305 can extend to a lesser extent than the second flexible portion 330 when the implantable medical device 12 is deployed from the distal tube 300. After deployment of the implantable medical device 12, the distal tube 300 can relax to a fourth length L4 that is the same as or about the same as the first length L1 prior to engaging the implantable medical device 12. The interference spiral cut features may limit the distal motion when deploying an implantable medical device 12.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 99%.

The descriptions contained herein are examples of embodiments of the invention and are not intended in any way to limit the scope of the invention. As described herein, the invention contemplates many variations and modifications of the inventive delivery and release system for a vascular occlusion device, including numerous configurations, numerous stiffness properties and methods for delivering the same. Also, there are many possible variations in the materials and configurations of the release mechanism. These modifications would be apparent to those having ordinary skill in the art to which this invention relates and are intended to be within the scope of the claims which follow.

## Claims

1. A detachment system comprising:
a generally hollow distal tube defining a longitudinal axis and comprising a distal end, a first flexible portion, and a second flexible portion comprising interference spiral cuts throughout and positioned between the first flexible portion and the distal end; and
an engagement system configured to engage an implantable medical device to the distal end of the distal tube and configured to deploy the implantable medical device from the distal end of the distal tube,
wherein the engagement system compresses the second flexible portion along the longitudinal axis to a greater extent than the first flexible portion when engaging the implantable medical device; and
wherein the second flexible portion is configured to extend along the longitudinal axis to a greater extent than the first flexible portion when the engagement system deploys the implantable medical device.

2. The detachment system of claim 1, wherein
the first flexible portion comprises interference spiral cuts having a first pitch; and
the second flexible portion comprises interference spiral cuts having a second pitch,
wherein the first pitch is different than the second pitch.

3. The detachment system of claim 1, wherein
the interference spiral cuts of the first and second flexible portions comprise about 240-degree cut sections and about 20-degree uncut sections.

4. The detachment system of claim 1,
wherein the distal tube measures a first length prior to the engagement system being engaged to the implantable medical device,
wherein the distal tube measures a second length when the engagement system is engaged to the implantable medical device, and
wherein a difference between the first length and the second length is between about 0.1 mm to about 0.25 mm.

5. The detachment system of claim 1,
wherein the distal tube measures a second length when the engagement system is engaged to the implantable medical device,
wherein the distal tube measures a third length that is a maximum length of the distal tube upon deployment of the implantable medical device by the engagement system, and
wherein a difference between the third length and the second length is between about 0.4 mm to about 0.8 mm.

6. The detachment system of claim 4,
wherein the distal tube measures a final length that is a net length of the distal tube when the first and second flexible portions of the distal tube are uncompressed, and
wherein a difference between the first length and the final length is between about 0 mm to about 0.5 mm.

7. The detachment system of claim 1, wherein the distal tube is under compression by a force of about 13 gf to about 26 gf when the engagement system is engaging the implantable medical device.

8. The detachment system of claim 7, wherein the force compresses the distal tube from about 0.15 mm to about 0.45 mm.

9. The detachment system of claim 6, wherein the engagement system further comprises:
a locking member; and
a loop wire,
wherein when the loop wire interacts with the locking member to engage the implantable medical device, a force on the loop wire moves the first and second flexible portions along a longitudinal axis to the second length from the first length of the distal tube when the loop wire engages the implantable medical device.

10. A detachment system comprising:
a generally hollow distal tube comprising:
a distal end;
a first flexible portion of a distal tube comprising interference spiral cuts having a first pitch; and
a second flexible portion of the distal tube comprising interference spiral cuts having a second pitch;
an engagement system configured to engage an implantable medical device at the distal end of the distal tube, the engagement system comprising:
a locking member;
a loop wire,
wherein when the loop wire interacts with the locking member to engage the implantable medical device, a force on the loop wire moves the first and second flexible portions along a longitudinal axis from a first length to a second length of the distal tube when the loop wire engages the implantable medical device; and
wherein the loop wire moves the second flexible portion along the longitudinal axis to a greater extent than the first flexible portion when engaging the implantable medical device.

11. The detachment system of claim 10, wherein a difference between the first length and the second length of the distal tube is between about 0.1 mm to about 0.25 mm.

12. The detachment system of claim 10,
wherein the engagement system is further configured to deploy the implantable medical device from the distal end of the distal tube; and
wherein the second flexible portion is configured to extend along the longitudinal axis to a greater extent than the first flexible portion when the engagement system deploys the implantable medical device.

13. The detachment system of claim 12,
wherein when the implantable medical device is deployed from the distal tube, an elastic force on the distal tube moves the first and second flexible portions along a longitudinal axis from a second length to a third length of the distal tube upon deployment of the implantable medical device by the loop wire; and
wherein a difference between the third length and the second length is between about 0.4 mm to about 0.8 mm.

14. The detachment system of claim 10, wherein the first pitch is different than the second pitch.

15. The detachment system of claim 2 or claim 14, wherein
the first pitch ranges from about 0.12 mm to about 0.18 mm; and
the second pitch ranges from about 0.08 mm to about 0.12 mm.

16. The detachment system of claim 10, further comprising:
a generally hollow proximal tube having a proximal end and a distal end; and
a coupling disposed inside the proximal end of the distal tube and inside the distal end of the proximal tube, joining the proximal and distal tubes.

17. A method comprising:
forming a first flexible portion and a second flexible portion on a distal tube between a proximal end of the distal tube and a distal end of the distal tube, the first and second flexible portions comprising interference spiral cuts;
engaging an implantable medical device with an engagement system;
extending the engagement system through the distal tube such that the implantable medical device is distal of the distal end of the distal tube;
fixing the engagement system to the distal tube; and
joining the proximal end of the distal tube to a distal end of a proximal tube.

18. The method of claim 17, further comprising:
forming the interference spiral cuts on the first flexible portion having a first pitch; and
forming the interference spiral cuts on the second flexible portion have a second pitch,
wherein the first pitch is different than the second pitch.

19. The method of claim 18, wherein
the second flexible portion is configured to extend along a longitudinal axis to a greater extent than the first flexible portion when the engagement system deploys the implantable medical device.
